# EUROPEAN PATENT SPECIFICATION

(11) **EP 4 115 947 B1**
(45) Date of publication and mention of the grant of the patent: **06.05.2026**
(21) Application number: 21763541.6
(22) Date of filing: 04.03.2021
(51) Int. Cl.: A61N 5/10

(54) **MEDICAL FIXING TOOL**
MEDIZINISCHES BEFESTIGUNGSWERKZEUG
OUTIL D'ATTACHE MÉDICAL

(30) Priority: 05.03.2020 JP 2020037443
(43) Date of publication of application: 11.01.2023
(73) Proprietor: Nihon Yamamura Glass Co., Ltd., Amagasaki-shi, Hyogo 660-8580 (JP); National University Corporation Kobe University, Kobe-shi, Hyogo 657-8501 (JP)
(72) Inventor: KIMURA, Shuji, Amagasaki-shi, Hyogo 6608580 (JP); OGURA, Shinichiro, Amagasaki-shi, Hyogo 6608580 (JP); MIYAWAKI, Daisuke, Kobe-shi, Hyogo 6578501 (JP); MUKUMOTO, Naritoshi, Kobe-shi, Hyogo 6578501 (JP); SASAKI, Ryohei, Kobe-shi, Hyogo 6578501 (JP); MIYAZAKI, Satoru, Kobe-shi, Hyogo 6578501 (JP)
(74) Representative: Manitz Finsterwald Patent- und Rechtsanwaltspartnerschaft mbB
(86) International application number: PCT/JP2021/008534
(87) International publication number: WO 2021/177421

(56) References cited:
- CN-A- 107 224 676
- JP-A- 2012 143 412
- JP-B2- 6 563 737
- US-A- 4 622 185
- US-A- 5 782 244
- US-A1- 2005 284 490
- US-A1- 2015 065 778
- US-A1- 2020 000 544

## Description

### Technical Field

The present invention relates to a medical immobilization device suitable for immobilizing a patient or subject (collectively referred to as a "patient" below) when performing, for example, radiotherapy or diagnostic imaging such as MRI.

In this specification, the term "up and down" for a patient lying on their back has a different meaning from up and down for a standing patient. The term "up and down" used here means that the side facing the patient's abdomen is "up" and the side facing the patient's back is "down."

### Background Art

IMRT (intensity-modulated radiation therapy) is one of the radiotherapies for cancer. IMRT divides the radiation applied to a patient from multiple directions into small beams and appropriately changes the irradiation shape of each beam to increase the irradiation dose to the tumor area, thereby improving the tumor control rate. IMRT also divides the irradiation time into several days (repeated irradiation) to minimize exposure to normal tissue and reduce complications.

IMRT uses a computer-controlled device that can adjust the spot of radiation irradiation with high precision. However, the treatment effect may be impaired or the risk of complications may be heightened, for example, if the patient under irradiation moves and the irradiation position shifts, or if the position of the patient under re-irradiation shifts. Thus, the patient under irradiation must be firmly immobilized.

Conventionally, a patient under irradiation is immobilized by using a top fixture and a bottom fixture that sandwich the patient lying on their back from the upper and lower directions. As shown in PTL 1, the top fixture is shaped by covering a patient lying on their back with a perforated resin mask that is softened beforehand by heating, by pressing it from above, and cooling to solidify it for conformity to the shape of the patient. For the bottom fixture, as shown in PTL 2, a suction mat is often used in the following manner. A patient is laid on their back on an airtight mat that contains beads and that is fixed to a treatment table, and air in the mat is removed to allow the mat to conform to the shape of the patient. To immobilize a patient, the two fixtures are connected so that the patient's body is tightly sandwiched from above and below.

Additionally, as shown in PTL 3, a fixture that does not use the above resin mask and suction mat is also proposed, which is produced by embedding a patient in frothed polyurethane foam before it softens.

### Citation List

### Patent Literature

PTL 1: Patent No. 6563737
PTL 2: Patent No. 6619609
PTL 3: JPH03-74592A

US 2005/0284490 A1 discloses a method and device for immobilization and registration. The device comprises a cantilevered support shell, a pod liner, a foam insert, a face mask and a facemask-to-shell attachment system.

Another device for immobilization is known from US5782244A.

### Technical Problem

Although a certain degree of reproducibility of the shape can be achieved when a patient lying on their back is immobilized from the upper side by using the method disclosed in PTL 1, the patient must bear a great mental and physical burden during production and treatment by the method. Immobilizing the body from the lower side as disclosed in PTL 2 is more likely to reduce such burdens. However, the method using a suction mat is unsatisfactory in immobilization performance due to its difficulty in reproducing the shape of the patient because of the complexity of the shape formation procedure coupled with the mat material. As noted above, conventional immobilization methods impose a great burden on the patient because the patient is immobilized mainly by being covered from above with a top fixture that reproduces the shape of the patient. Conventional immobilization methods also have a decreased overall immobilization precision due to the lack of shape reproducibility precision by the bottom fixture.

Additionally, in the series of the fixtures proposed by PTL 3, the following problems arise: the fixtures impose a great burden on the patient because it is necessary to restrain the movement of the patient until the foam solidifies; production of the fixtures is very complicated and places a heavy burden on the person who produces them; and the strength of the completed foam fixture is weak (brittle), and breaks during a series of treatments.

### Summary of the Invention

An object of the invention is to provide a medical immobilization device that is capable of firmly and precisely immobilizing a patient and is easy to use.

### Solution to Problem

The above object is accomplished by the features of claim 1.

A medical immobilization device has the features of claim 1. It is configured to immobilize a specific site of a patient with the patient lying on their back. The medical immobilization device comprises a block made of a material that allows radiation to pass through. The block comprises a holder portion in which at least a region from the lower surface to both sides of the specific site tightly fits and has all or a part of the upper side thereof open.

In a preferable embodiment, the upper edge of a portion of the holder portion that comes into contact with the sides of the specific site is located above the center of the specific site in the up-and-down direction.

In a preferable embodiment, the region from the lower surface to both sides of the specific site fits in the holder portion with the entire surface of the region being tightly in contact.

The holder portion may comprise a ventilation portion with which the specific site does not come into contact, and the region from the lower surface to both sides of the specific site tightly fits in the holder portion excluding the ventilation portion.

The block comprises a head immobilization part for immobilizing the head, a shoulder immobilization part for immobilizing the shoulders, and at least either a jaw immobilization part for immobilizing the jaw or a forehead immobilization part for immobilizing the forehead, or both. The head immobilization part, the shoulder immobilization part, and at least either the jaw immobilization part for immobilizing a jaw or the forehead immobilization part for immobilizing a forehead, or both, are connected to each other. The head immobilization part comprises at least a right-side head immobilization part for immobilizing the right- side head and a left-side head immobilization part for immobilizing the left-side head.

At least either the jaw immobilization part or the forehead immobilization part, or both, are connected at each end to the right-side head immobilization part and the left-side head immobilization part across the upper open portion between the right-side head immobilization part and the left-side head immobilization part.

In a preferable embodiment, the block comprises the head immobilization part, the shoulder immobilization part, and the jaw immobilization part, the head immobilization part, the shoulder immobilization part, and the jaw immobilization part being connected to each other,
the head immobilization part comprises a right temporal region immobilization part for immobilizing the right temporal region, a left temporal region immobilization part for immobilizing the left temporal region, and an occipital region immobilization part for immobilizing the occipital region,
the occipital region immobilization part and the shoulder immobilization part are connected in the longitudinal direction,
the left temporal region immobilization part and the
   right temporal region immobilization part are respectively connected to the left side and the right side of the occipital region immobilization part, and
the jaw immobilization part is connected at each end to the left temporal region immobilization part and the right temporal region immobilization part across the upper open portion between the left temporal region immobilization part and the right temporal region immobilization part.

In another preferable embodiment, the block comprises the head immobilization part, the shoulder immobilization part, the jaw immobilization part, and the forehead immobilization part, the head immobilization part, the shoulder immobilization part, the jaw immobilization part, and the forehead immobilization part being connected to each other,
the head immobilization part and the shoulder immobilization part are connected in the longitudinal direction, and
the jaw immobilization part and the forehead immobilization part are connected at each end to the left head immobilization part and the right head immobilization part across the upper open portion between the right-side head immobilization part and the left-side head immobilization part.

### Advantageous Effects of Invention

The present invention provides a medical immobilization device that is capable of firmly immobilizing a patient; that contributes to the improvement of immobilization precision, reproducibility, and workability for immobilization; and that also contributes to reducing the burden on the patient.

The medical immobilization device in a preferable embodiment firmly immobilizes the specific site of a patient by the holder portion that has a depth of a half or more of the thickness of the specific site, and also improves immobilization precision and reproducibility due to immobilization by the deep holder portion. Additionally, the mental and physical burden on the patient is reduced by eliminating (or minimizing) the immobilization of the patient lying on their back from above, and mainly by immobilizing the patient from below by the holder portion.

The medical immobilization device in a preferable embodiment makes it easier for the block to have a high degree of flexibility in shape due to the structure of the block composed of a plurality of parts.

The medical immobilization device in a more preferable embodiment can immobilize the head by the occipital region immobilization part and the temporal region immobilization parts and can immobilize from above at least either the jaw or the forehead of the patient by comprising at least either the jaw immobilization part or the forehead immobilization part, thereby firmly immobilizing the head and the neck of the patient (specific site) without covering the patient from above.

### Brief Description of Drawings

Fig. 1 is an illustration of a radiotherapy machine in which the medical immobilization device of the present invention is used.
Fig. 2 is a plane view of an embodiment of the present disclosure.
Fig. 3 is a side view of the embodiment shown in Fig. 2.
Fig. 4(A) is a sectional view taken along line A-A in Fig. 2.
Fig. 4(B) is a sectional view taken along line B-B in Fig. 2.
Fig. 4(C) is a sectional view taken along line C-C in Fig. 2.
Fig. 5 is a sectional view taken along line D-D in Fig. 2.
Fig. 6(A) and Fig. 6(B) are illustrations showing a state in which a patient's head and shoulders are immobilized by using the embodiment shown in Fig. 2.
Fig. 7 is a plane view showing another embodiment of the present disclosure.
Fig. 8 is a side view of the embodiment shown in Fig. 7.
Fig. 9 is illustrations showing a state in which a patient's head and shoulders are immobilized by using the embodiment shown in Fig. 7.
Fig. 10 is an illustration showing a production method for the medical immobilization device of the present invention.
Fig. 11 is views showing another embodiment of the present disclosure. Fig. 11(A) is a plane view, and Fig. 11(B) is a sectional view taken along line E-E.
Fig. 12(A) is a view showing a basic shape for use in the production of the embodiment shown in Fig. 11. Fig. 12(B) is an illustration showing a state in which a patient's head and shoulders are immobilized by using the embodiment produced from the basic shape of Fig. 12(A).
Fig. 13 is a plane view showing another embodiment of the present disclosure .
Fig. 14 is a sectional view taken along line F-F in Fig. 13.
Fig. 15 is an illustration showing a state in which a patient's head and shoulders are immobilized by using the embodiment shown in Fig. 13.
Fig. 16 is a plane view showing another embodiment of the present invention.
Fig. 17 is a side view of the embodiment shown in Fig. 16.
Fig. 18 is an exploded perspective view of the embodiment shown in Fig. 16.
Fig. 19(A) is a perspective view showing a left temporal region immobilization part. Fig. 19(B) is a perspective view showing an occipital region immobilization part. Fig. 19(C) is a perspective view showing a jaw immobilization part.
Fig. 20 is an illustration showing a state in which a patient's head and shoulders are immobilized by using the embodiment shown in Fig. 16.
Fig. 21 is a plane view of another embodiment of the present invention.
Fig. 22 is a side view of the embodiment shown in Fig. 21.
Fig. 23 is an exploded perspective view of the embodiment shown in Fig. 21.
Fig. 24 is an illustration showing a state in which a patient's head and shoulders are immobilized by using the embodiment shown in Fig. 21.

### Description of Embodiments

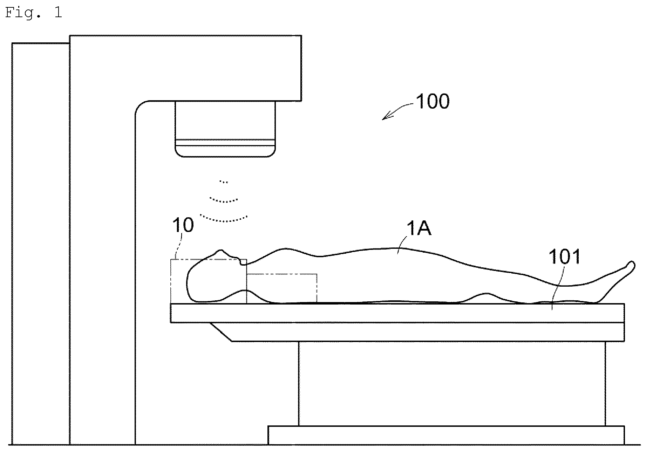

As shown in Fig. 1, a medical immobilization device 10 according to the present invention is used on a support table 101 by which a patient 1A lying on their back is supported in a radiotherapy equipment 100. The medical immobilization device 10 is positioned and immobilized at a predetermined position on the support table 101 by a holding mechanism (not shown). Embodiments of the present invention will be described with reference to drawings.

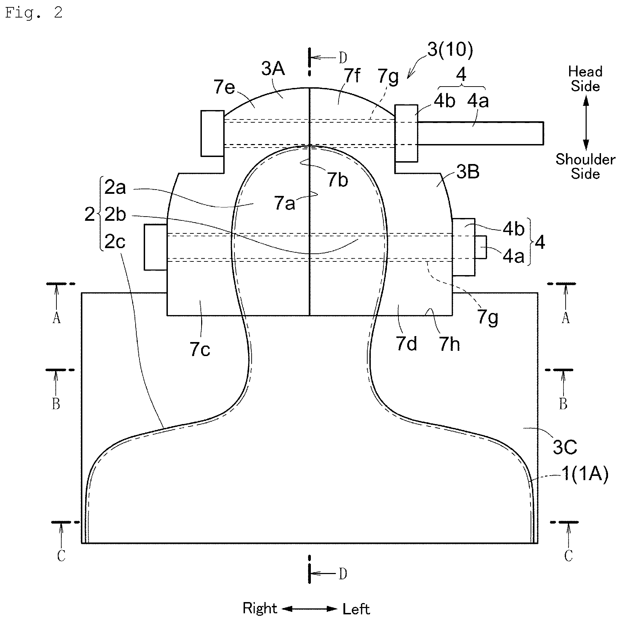

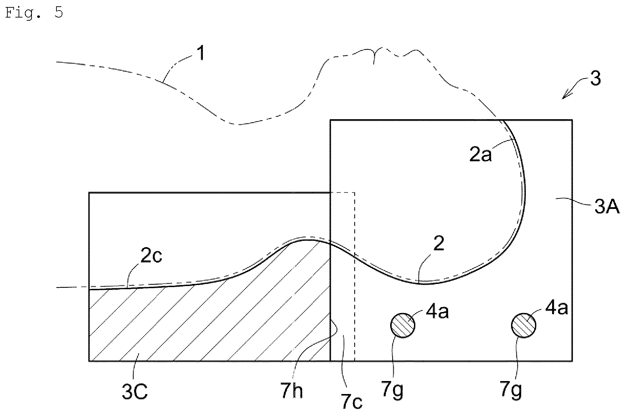

The medical immobilization device 10 shown in Figs. 2 to 5, is for immobilizing the specific site 1 of the body of the patient 1A, for example, during radiation in IMRT, and is formed of a block 3 made of a material that allows radiation to pass through. The block 3 has appropriate elasticity, and comprises a holder portion 2 into which at least a region from the lower surface to both sides of the specific site 1 of the body of the patient 1A lying on their back fits with the entire surface of the region being tightly in contact. In the holder portion 2, at least a portion of the upper edge 2d of the portion that comes into contact with the sides of the specific site 1 is located above the center of the specific site 1 in the up-and-down direction.

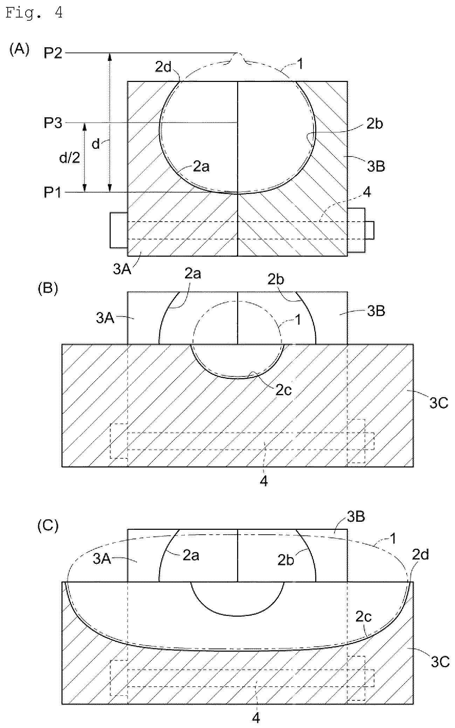

The phrase "the center of the specific site 1 in the up-and-down direction" as used here means, as shown in Fig. 4(A), a height position P3 that is a half of the maximum height of the specific site 1 of the patient 1A in a natural posture lying on a flat plane P1, i.e., height d that is from the plane P1 to a top P2 (for example, the top of the nose in the case of the head).

Preferably, at least a portion of the upper edge 2d of the portion of the holder portion that comes into contact with the sides of the specific site 1 falls within the range of two-fifths of the height d that goes downward from the top P2 of the specific site 1. This immobilizes the specific site 1 more reliably. In the embodiments shown in Figs. 2 to 5, the entire upper edge 2d of both sides of the portion of the holder portion 2 that come into contact with the head is located above the height position P3 that is in the center of the specific site 1 in the up-and-down direction.

The block 3 has all or part of the upper side open so as not to cover the upper side of the patient. The block 3 is preferably structured so as to have the upper part of the specific site 1 mostly open when the specific site 1 fits in the holder portion 2.

The medical immobilization device 10 of the present embodiment is intended to firmly immobilize mainly the head and neck of the patient 1A in a natural posture lying on their back. In the medical immobilization device 10 of the present embodiment, the region from the head to the shoulders of the patient 1A is the specific site 1, and at least the lower side fits in the holder portion 2.

In the following embodiments, the specific site 1 means, but is not limited to, the head, neck, and shoulders of the patient 1A. A "shoulder" is the upper portion of the body part at which the arm is connected to the torso, and the part from there to the base of the neck. The "neck" is the part between the head and shoulders. The "head" is the part above the neck and is divided into the occipital region that is the lower part; the left and right temporal regions, which are the left and right side parts; the parietal region, which is the top part of the head; the jaw part; and the forehead part.

Unless otherwise defined, the "left-right direction" refers to the left-right direction as seen from the patient 1A lying on their back on the support table 101, and the "up-and-down direction" refers to the direction between the ventral side (upward direction) and the dorsal side (downward direction) of the patient lying on their back on the support table 101. The "longitudinal direction" refers to the direction perpendicular to the up-and-down direction or the left-right direction. The direction toward the head of the patient 1A is the head side, and the direction opposite the head side is the shoulder side. The terms "thickness" and "height" refer to the dimension (distance) in the up-and-down direction, and the term "width" refers to the dimension (distance) in the left-right direction. The term "length" refers to the dimension (distance) in the longitudinal direction.

The thus-structured medical immobilization device 10 of this embodiment can firmly immobilize the specific site 1 of the patient 1A by, so to speak, the holder portion 2 with a depth of a half or more of the thickness of, in particular, the head of the specific site 1. Immobilization by using the deep holder portion 2 can also improve immobilization precision and reproducibility.

Additionally, when all or part of the upper side of the block 3 is released allowing the patient 1A lying on their back to not (or barely) be immobilized from the upper side but be only immobilized by the holder portion 2 from the lower side, the mental and physical burden on the patient 1A can be reduced. This effect is particularly useful for a claustrophobic patient 1A because the entire facial area is not covered by the block 3.

Moreover, the immobilization of the specific site 1 of the patient 1A is basically complete only when the specific site 1 fits in the holder portion 2. Thus, the medical immobilization device 10 of this embodiment can also improve the workability of immobilization.

The block 3 is made from a material that allows radiation to pass through. Such materials include foamed resin materials such as urethane foam and Styrofoam, carbon materials (CFRP), and wood. From the standpoint of ease of processing, foamed resin materials are particularly preferable.

The block 3 may be formed of a single component, or may be formed of a plurality of parts (block parts) that can be connected to each other, for example, for the purpose of having a high degree of flexibility in shape of the block 3.

In this embodiment, as shown in Fig. 2, the block 3 is composed of two parts for immobilizing the head (i.e., a right-side head immobilization part 3A for immobilizing the right part of the head and a left-side head immobilization part 3B for immobilizing the left part of the head) and a shoulder immobilization part 3C for immobilizing the shoulders, which are all connected. The neck of the patient 1A may be immobilized by the head immobilization parts or the shoulder immobilization part 3C. The neck portion near the shoulders may be immobilized by the shoulder immobilization part 3C, and the neck portion near the head may be immobilized by the head immobilization parts.

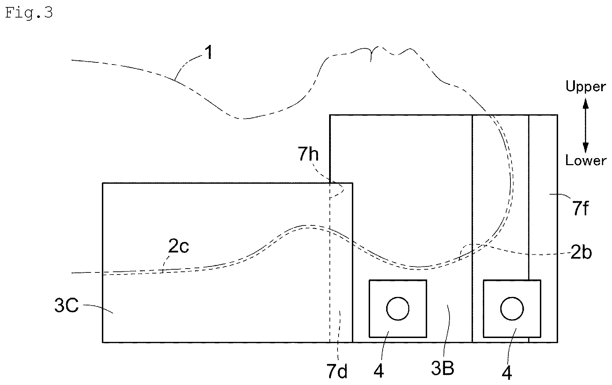

The right-side head immobilization part 3A and the left-side head immobilization part 3B are joined by the respective inner facing surfaces 7a and 7b being butted. As shown in Figs. 3 to 5, the right-side head immobilization part 3A and the left-side head immobilization part 3B have a greater thickness than for the shoulder immobilization part 3C. As shown in Fig. 4(A), intercommunicable holder portions 2a, 2b are formed on each upper surface of the right-side head immobilization part 3A and the left-side head immobilization part 3B. The holder portions 2a and 2b have a shape along by the region from the lower side to the respective sides of the head of the patient 1A, and this region of the head tightly fits in the holder portions 2a and 2b.

Although the right-side head immobilization part 3A and the left-side head immobilization part 3B have a symmetrical shape, the shape of the specific site 1 of the patient 1A is not always symmetrical. Thus, the holder portions 2a, 2b do not necessarily have a symmetrical shape.

The right-side head immobilization part 3A and the left-side head immobilization part 3B respectively comprise a mating portion 7c and a mating portion 7d at each shoulder-side end at which the right-side head immobilization part 3A and the left-side head immobilization part 3B fit the shoulder immobilization part 3C. On the opposite side of the mating portions 7c, 7d, the right-side head immobilization part 3A and the left-side head immobilization part 3B respectively comprise crown portions 7e, 7f, which are smaller in width than the mating portions 7c, 7d.

As shown in Figs. 2, 3, and 4(A), the right-side head immobilization part 3A and the left-side head immobilization part 3B are connected and immobilized by two connectors 4. The connectors 4 are each composed of a bolt 4a and a nut 4b. Through-holes 7g for the bolts 4a to be inserted are present at two points in the lower part of each of the holder portions 2a, 2b of the right-side head immobilization part 3A and the left-side head immobilization part 3B and the lower part of the crown portions 7e, 7f.

As shown in Figs. 3, 4(B), 4(C), and 5, the shoulder immobilization part 3C comprises a holder portion 2c on the upper surface. The holder portion 2c has a shape along by the region from the lower part of the neck and the shoulders to both sides of the patient 1A, and the region of the neck and shoulders tightly fits in the holder portion 2c. The shoulder immobilization part 3C comprises at an end thereof closer to the head a recess 7h, into which the mating portions 7c, 7d of the right-side head immobilization part 3A and the left-side head immobilization part 3B fit. The right-side head immobilization part 3A, the left-side head immobilization part 3B, and the shoulder immobilization part 3C are connected and immobilized by the mating portions 7c, 7d of the parts 3A, 3B tightly mating with the recess 7h of the part 3C.

With the right-side head immobilization part 3A and the left-side head immobilization part 3B connected to the shoulder immobilization part 3C, the holder portions 2a, 2b, 2c of the parts 3A, 3B, and 3C become intercommunicable to thereby form a recessed portion (space) for supporting the specific site 1 (i.e., the holder portion 2). The head in the specific site 1 is supported while being sandwiched by the right-side head immobilization part 3A and the left-side head immobilization part 3B from left and right. The region from the neck to the shoulders are supported by the shoulder immobilization part 3C.

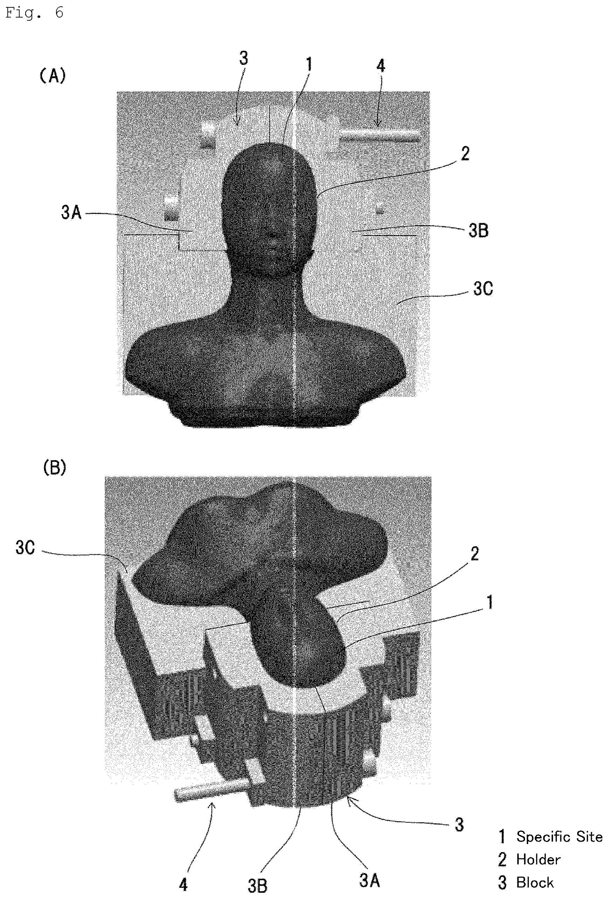

Figs. 6(A) and 6(B) show that the head, neck, and shoulders (specific site 1) of the patient 1A lying on their back are immobilized by the medical immobilization device 10. The region from the lower part to both sides of the head of the patient 1A tightly fits in the holder portions 2a, 2b of the right-side head immobilization part 3A and the left-side head immobilization part 3B. The region from the lower part to both sides of the neck and shoulders of the patient 1A tightly fits in the holder portion 2c of the shoulder immobilization part 3C. This allows the medical immobilization device 10 to firmly immobilize the head, neck, and shoulders of the patient 1A with the upper part open.

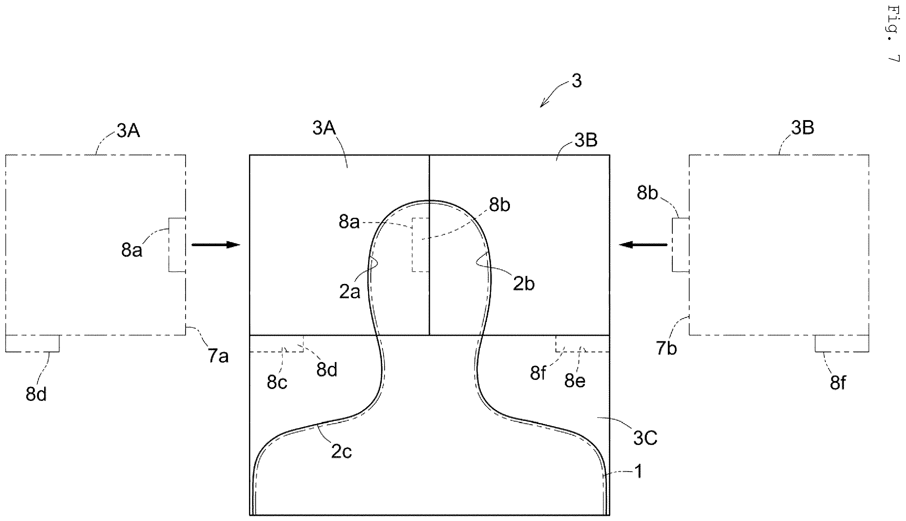

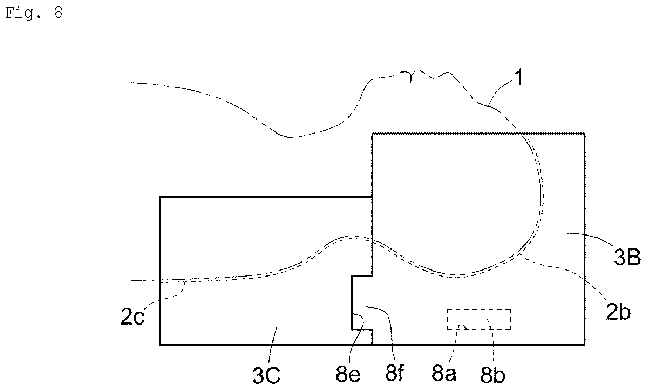

Figs. 7 and 8 show another embodiment of the present disclosure.

In this embodiment, in place of the connectors 4, an engagement recess 8a is formed in one of the facing surfaces 7a, 7b of the right-side head immobilization part 3A and the left-side head immobilization part 3B, and an engagement protrusion 8b that engages with the engagement recess 8a is formed in the other of the facing surfaces 7a, 7b to allow the linkage between the right-side head immobilization part 3A and the left-side head immobilization part 3B. In this embodiment, an engagement recess 8c is formed in one of the facing surfaces of the right-side head immobilization part 3A and the shoulder immobilization part 3C, and an engagement protrusion 8d is formed in the other to allow the linkage between the parts 3A and 3C. In the same manner, an engagement recess 8e is formed in one of the facing surfaces of the left-side head immobilization part 3B and the shoulder immobilization part 3C, and an engagement protrusion 8f is formed in the other to allow the linkage between the parts 3B and 3C.

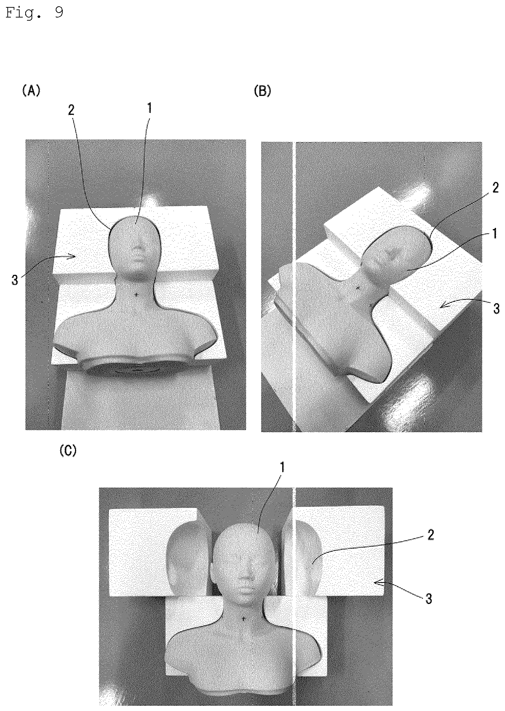

Fig. 9 shows that the head, neck, and shoulders (specific site 1) of the patient 1A lying on their back are immobilized by the medical immobilization device 10 shown in Figs. 7 and 8. The region from the lower part to both sides of the head of the patient 1A tightly fits in the holder portions 2a, 2b of the right-side head immobilization part 3A and the left-side head immobilization part 3B. The region from the lower part to both sides of the neck and shoulders of the patient 1A tightly fits in the holder portion 2c of the shoulder immobilization part 3C. This allows the medical immobilization device 10 to firmly immobilize the head, neck, and shoulders of the patient 1A with the upper part open.

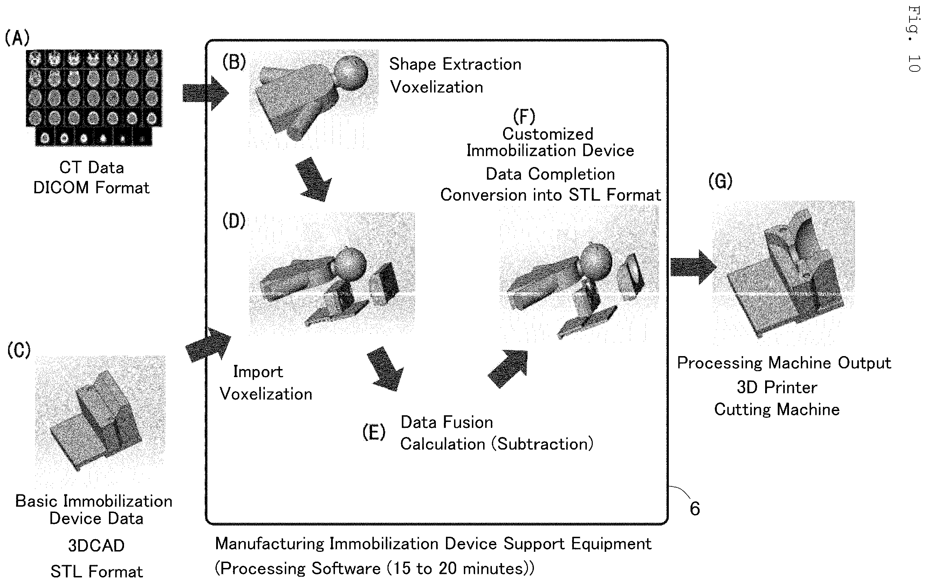

The medical immobilization device 10 of the present invention is produced by sequentially performing the steps shown in Fig. 10. Performing the method shown in Fig. 10 provides the block 3 comprising the holder portion 2 in which at least the region from the lower part to both sides of the specific site 1 tightly fits. Although the medical immobilization device 10 shown in Fig. 10 is different from the medical immobilization device 10 described in the embodiments above, any medical immobilization device 10 described in the embodiments of the present disclosure above or in the embodiments described later can be produced by performing the steps shown in Fig. 10.

Step (1): First, as shown in Figs. 10(A) and 10(B), medical image data (DICOM format) such as CT data or MRI data of the patient 1A are input to a manufacturing immobilization device support equipment 6 incorporating processing software (indicated by a rectangular frame in the figure). The manufacturing immobilization device support equipment 6 executes a shape extraction voxelization program to extract the shape (outer shape) of the specific site 1 of the body of the patient 1A from the medical image data and to perform voxelization. In this embodiment, because the specific site 1 of the body of the patient 1A is immobilized by the three parts (the right-side head immobilization part 3A, the left-side head immobilization part 3B, and the shoulder immobilization part 3C), the extraction and voxelization of the outer shape of the specific site 1 are performed for each of the three regions comprising the regions immobilized by the parts 3A, 3B, and 3C.

The medical image data is input by using appropriate data entry means. When the patient 1A undergoes IMRT, a medical image such as CT data is usually obtained beforehand in the hospital, and it thus makes sense to use such image data. Alternatively, other image data from which the body shape of the patient 1A can be extracted (e.g., images obtained with a human body scanner) may be used instead. The manufacturing immobilization device support equipment 6 comprises a data input/output unit, a storage unit, a display unit, an operation unit, and a control unit including a CPU for executing a predetermined program. The processing software is stored in the storage unit.

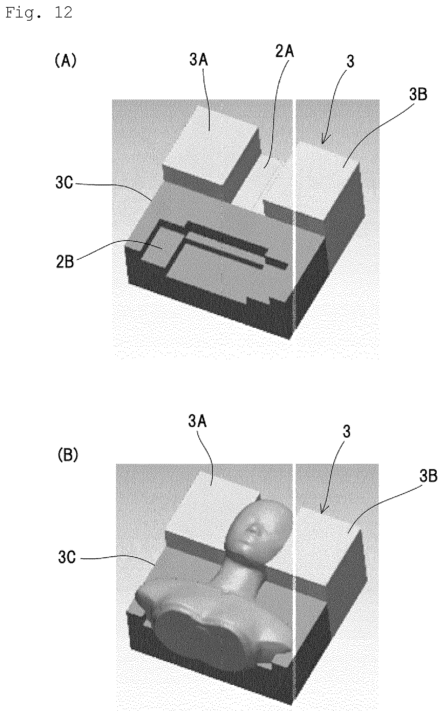

Step (2): As shown in Fig. 10(C), basic shape data (e.g., data in STL format such as 3D CAD) of the medical immobilization device 10 in this embodiment are input (imported) to the manufacturing immobilization device support equipment 6. The manufacturing immobilization device support equipment 6 executes a voxelization program to perform voxelization of the basic shape data. This step (2) may be performed, for example, before step (1). The basic shape data refer to 3D data illustrating the outer shape of the plurality of parts of the block 3 before the holder portion 2 is formed. In this embodiment, the basic shape data are 3D data of the outer shape corresponding to the right-side head immobilization part 3A, the left-side head immobilization part 3B, and the shoulder immobilization part 3C. Fig. 12(A), which is explained later, shows a basic shape for use in the production of the embodiment shown in Fig. 12(B). In Fig. 12(A), the same reference numerals as those of the parts of the block 3 that has the holder portion 2 formed are also added to the plurality of the parts.

Step (3): After steps (1) and (2), as shown in Figs. 10(D) and 10(E), the manufacturing immobilization device support equipment 6 executes a data fusion operation program and subtracts the shape of the patient 1A from the basic shape data of the medical immobilization device 10 to obtain the shape of the holder portion 2, thereby completing 3D shape data for each part of the medical immobilization device 10 tailored for the body shape of the patient 1A. Specifically, in this embodiment, the suitable shape of the holder portion 2 varies depending on the body shape of the patient 1A, and the shape of the holder portion 2 that matches the body shape of the patient 1A is given in the completed shape data. Of course, in this customization, the shape of the portion other than the holder portion 2 in the medical immobilization device 10 may be appropriately adjusted.

Step (4): After step (3), as shown in Figs. 10(F) and 10(G), the completed shape data are converted into an appropriate format (e.g., STL format) and output to a processing machine (e.g., a cutting machine or a 3D printer), which is not shown, followed by producing the medical immobilization device 10 by using the processing machine. The processing machine for use may be, for example, a machine that cuts out a medical immobilization device 10 of a predetermined shape from a block made of Styrofoam; or, for example, a machine that performs additional processing that laminates a CFRP material to form a medical immobilization device 10 of a predetermined shape. Each part of the medical immobilization device 10 may also be produced by using a processing machine. For example, only some parts of the medical immobilization device 10 may be produced; e.g., only the block 3 is produced with a processing machine, and the other parts such as the connectors 4 for use are ready-made products. Production time for the block 3 comprising a plurality of parts can be shortened by simultaneously producing separate parts with multiple processing machines.

In the conventional method for immobilizing the patient 1A by using the top fixture and bottom fixture described above, for example, a radiological technologist produces the top fixture and the bottom fixture on site in many cases. However, this production takes time and effort, and this also imposes a burden on the patient 1A. Additionally, this method also has a drawback in that the patient 1A must endure heat when the face is covered with a mask that has been heated and softened during the production of the top fixture.

However, the medical immobilization device 10 of this embodiment does not require that a person such as a radiological technologist perform the above-mentioned steps (1) to (4) for the production of the medical immobilization device 10 on site. The medical immobilization device 10 of this embodiment can be produced beforehand, for example, by outsourcing its production before treatment, and the patient 1A also does not have to endure suffering. These can lead to shortened treatment time or reduced burden on the patient 1A.

Additionally, in the conventional immobilization method, immobilization may become insufficient depending on the operator's skill level in producing the fixtures, and an unintended clearance may form between the patient 1A and the fixture. Moreover, if the suction mat, which is currently used as the bottom fixture, is deformed due to an air leak, the mat will no longer conform to the body shape of the patient 1A, and IMRT treatment cannot be continuously performed.

In contrast, the medical immobilization device 10 in this embodiment does not require work skill because the production of the block 3 is mechanized, and the completed medical immobilization device 10 perfectly fits the body shape of the patient 1A, improving immobilization precision from the current levels, and preventing problems such as air leak.

It goes without saying that the present invention is not limited to the above embodiments, and can be modified in various ways without departing from the scope of the present invention as defined in the appended claims. For example, the following modifications can be made.

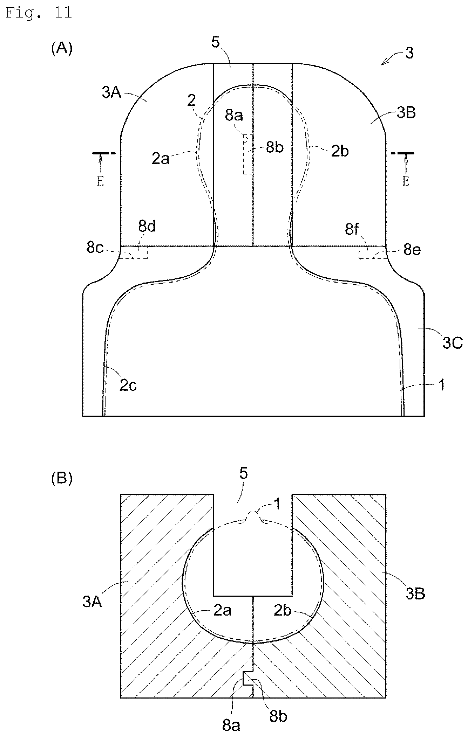

In the embodiments above, each holder portion 2 is provided so as to surround the head of the patient 1A lying on their back from three directions. However, the holder portion 2 is not limited to these embodiments. As in the embodiments shown in Figs. 11 and 12(B), the right-side head immobilization part 3A and the left-side head immobilization part 3B of the block 3 may be structured such that the top portion of the head is opened to provide a ventilation section 5, and such that the ventilation section 5 serves as an escape route for the hair.

In the embodiments above, the holder portion 2 has a structure in which the region from the head to the shoulders of the patient 1A fits. However, the holder portion 2 is not limited to this structure. The holder portion 2 may be structured such that only the head and the shoulders of the patient 1A fit in, and such that the neck is only placed on the upper surface of the shoulder immobilization part 3C. In this manner, the shape and arrangement of the holder 2 can be altered in various ways.

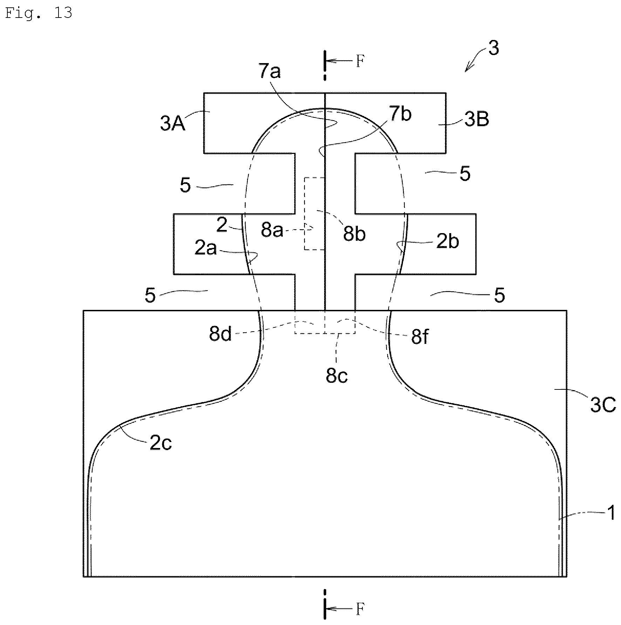

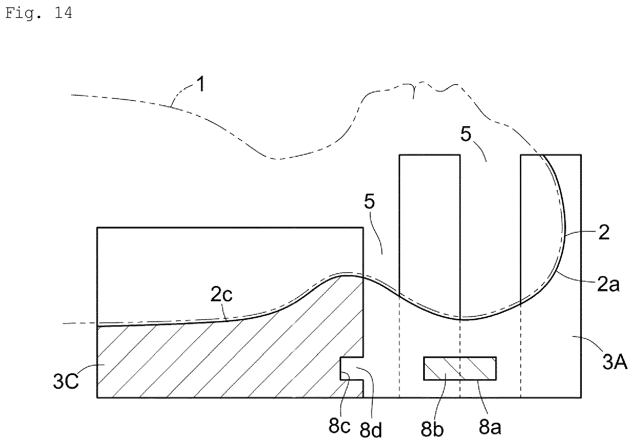

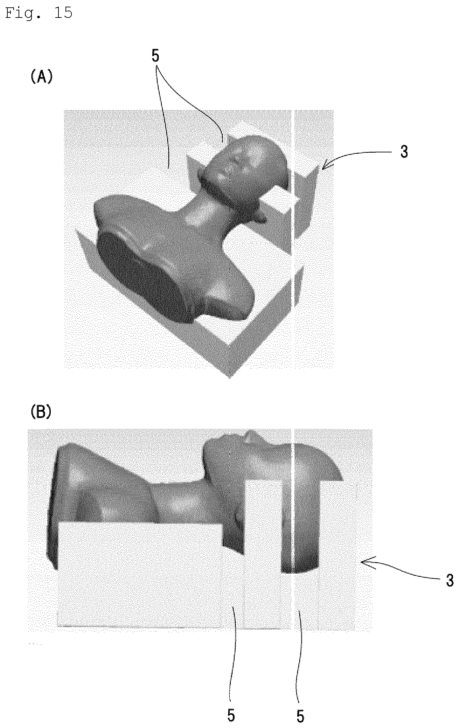

In the embodiments above, the holder portion 2 is structured such that the specific site 1 of the patient 1A (i.e., the region from the lower part to both sides of the head, neck, and shoulders) fits in the holder portion 2 with the entire surface of the region being tightly in contact. However, in the embodiment shown in Figs. 13 to 15, the holder portion 2 is structured not to come in contact with some region of the specific site 1 of the patient 1A. In this embodiment, as shown in Fig. 14, the region from the lower part to both sides of the head, neck, and shoulders fits in the holder portion 2, excluding the ventilation portion 5.

In the embodiment shown in Figs. 13 to 15, each end on the shoulder side of the right-side head immobilization part 3A and the left-side head immobilization part 3B is notched from the side to the top, leaving the lower portion. The middle portion closer to the top of the head of the right-side head immobilization part 3A and the left-side head immobilization part 3B is notched from both sides toward the top, leaving the lower portion. With the right-side head immobilization part 3A, the left-side head immobilization part 3B, and the shoulder immobilization part 3C being joined, the ventilation portion 5 is formed by a space between the right-side head immobilization part 3A and the shoulder immobilization part 3C and a space between the left-side head immobilization part 3B and the shoulder immobilization part 3C. Moreover, the ventilation portion 5 is also formed by the notch of the right-side head immobilization part 3A and the head-side notch of the left-side head immobilization part 3B.

In the embodiment of Figs. 13 to 15, one of the facing surfaces 7a and 7b of the right-side head and left-side head immobilization parts 3A, 3B has the engagement recess 8a, and the other has the engagement recess 8b that engages with the engagement recess 8a, allowing the linkage between the parts 3A and 3B. In this embodiment, the engagement recess 8c is formed in the center of the left-right direction of the plane of the shoulder immobilization part 3C facing the head immobilization parts 3A, 3B, and the engagement protrusions 8d, 8f are formed on the planes of the right-side and left-side head immobilization parts 3A, 3B, which are facing the shoulder immobilization part 3C. The engagement of the engagement protrusions 8d, 8f with the engagement recess 8c allows for the linkage between the part 3A and part 3C and between the part 3B and the part 3C.

Generally, for example, the cheeks of the patient 1A are soft. Even if the holder portion 2 supports such portions, the effect of immobilizing the body of the patient 1A is hardly obtained. Additionally, an immobilization device may not be attached due to changes in the body shape of the patient 1A caused by, for example, edema, during treatment. In this way, the body of the patient 1A is expected to have a plurality of sites in which an immobilization effect cannot be achieved. Additionally, if the holder portion 2 and the patient 1A are in contact with each other in many regions, air permeability decreases, and the patient 1A easily perspires, feeling increased discomfort.

In the embodiment shown in Figs. 13 to 15, the ventilation part 5 is formed in the block 3 to the extent that the ventilation part 5 does not hinder the immobilization of the body. This saves materials, reduces manufacturing costs, shortens the working time if additional processing is performed with a processing machine in the step of Fig. 10(G), and also shortens the working time in cutting with a processing machine if the material to be cut is provided with the ventilation portion 5 from the beginning.

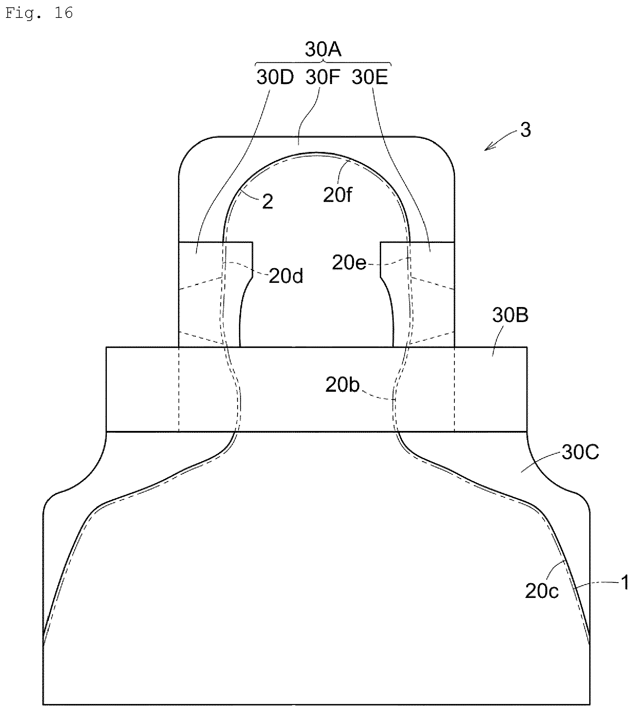

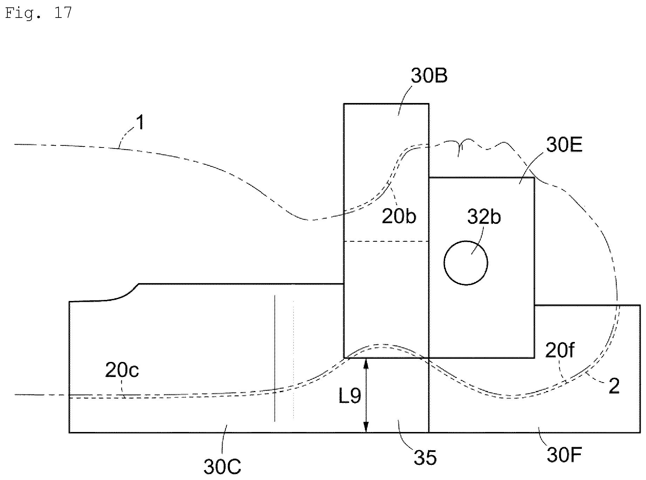

Figs. 16 to 20 show yet another embodiment in which the block 3 comprises a head immobilization part 30A, a shoulder immobilization part 30C, and a jaw immobilization part 30B, which are connected to each other. The head immobilization part 30A comprises a right temporal region immobilization part 30D for immobilizing the right temporal region of the head, a left temporal region immobilization part 30E for immobilizing the left temporal region, and an occipital region immobilization part 30F for immobilizing the occipital region. In the embodiment of Fig. 16, the neck is immobilized by the shoulder immobilization part 30C at the part closer to the shoulders and by the head immobilization part 30A at the part closer to the head. However, the neck may be immobilized by either the head immobilization part 30A or the shoulder immobilization part 30C.

As shown in Fig. 16, the occipital region immobilization part 30F and the shoulder immobilization part 30C are connected in the longitudinal direction. The right and left temporal region immobilization parts 30D and 30E are each on the corresponding side of and connected to the occipital region immobilization part 30F. The jaw immobilization part 30B is connected at each end to the right and left temporal region immobilization parts 30D and 30E across the upper open portion between the right and left temporal region immobilization parts 30D and 30E. The jaw immobilization part 30B covers only a portion of the upper open part of the block 3, with the majority left open.

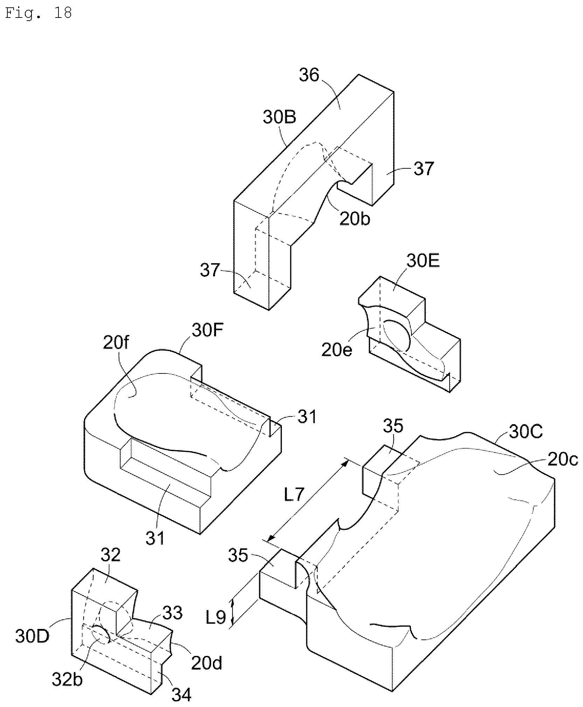

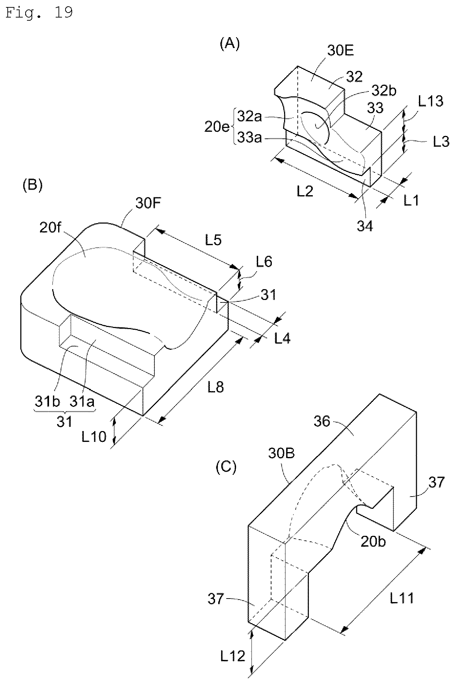

As shown in Figs. 18 and 19(B), the occipital region immobilization part 30F has a substantially rectangular parallelepiped outer shape and comprises a holder portion 20f on the upper surface. The holder portion 20f has a shape along by the lower surface region of the head and neck from the head to the neck of the specific site 1 of the patient 1A, and this region of the head and neck can fit in the holder portion 20f. The region of the specific site 1 that fits in the holder portion 20f is not strictly limited to the region along the lower surface of the head and neck. The region around it (e.g., a portion of the sides of the head or a portion of the top of the head) may fit in the holder portion 20f. The occipital region immobilization part 30F comprises a stepped portion 31 along by the longitudinal direction at each end of the upper surface in the width direction. The right and left temporal region immobilization parts 30D and 30E lock on the corresponding stepped portion 31. The stepped portions 31 each comprise a side surface 31a and a bottom surface 31b.

As shown in Figs. 18 and 19(A), the right and left temporal region immobilization parts 30D and 30E are each integrally formed so as to comprise a temporal portion 32 for immobilizing a temporal region, a neck portion 33 for immobilizing the neck, and an engagement portion 34 that is continuous with the lower edge of the temporal portion 32 and the neck portion 33 and that engages with the corresponding stepped portion 31 of the occipital region immobilization part 30F. A holder portions 32a, 33a are formed on each mutually facing inner surface of the temporal portion 32 and the neck portion 33 of the right and left temporal region immobilization parts 30D and 30E. In the right and left temporal region immobilization parts 30D and 30E, the holder portions 32a and 33a communicate to form holder portions 20d, 20e. The right and left temporal region immobilization parts 30D and 30E have a symmetrical outer shape in the left-right direction. However, because the shape of the temporal regions and the neck of the patient 1A is not necessarily symmetrical in the left-right direction, the shape of the holder portions 20d, 20e is not limited to a symmetrical shape.

The holder portions 20d, 20e have a shape along by the region of the left or right side of the head and the left or right side of the neck from the head to the neck of the patient 1A. These regions of the head and neck can fit in the holder portions 20d, 20e. A through-hole 32b penetrating through the temporal portion 32 from the inner surface to the outer surface is located at the holder portion 32a. The through-hole 32b is located at a portion corresponding to the position of an ear of the patient 1A who is immobilized at the head by the right and left temporal region immobilization parts 30D and 30E.
Each through-hole 32b prevents the ear from being pressed and the generation of unpleasant resonance.

The right and left temporal region immobilization parts 30D and 30E are set to have a length shorter than the length of the occipital region immobilization part 30F. When the engagement portion 34 is locked on the stepped portion 31 of the occipital region immobilization part 30F, the right and left temporal region immobilization parts 30D and 30E are positioned closer to the shoulder side of the occipital region immobilization part 30F. This allows the top region of the head of the patient 1A to be opened without being covered by the block 3, secures ventilation, and reduces the discomfort felt by the patient 1A.

The width L1, length L2, and height L3 of the engagement portion 34 of the right and left temporal region immobilization parts 30D and 30E are set to be substantially the same as the width L4, length L5, and height L6 of the stepped portion 31 of the occipital region immobilization part 30F.

A holder portion 20c on the upper surface is formed at the shoulder immobilization part 30C. The holder portion 20c has a shape along by the region from the lower side to both sides of the shoulders of the patient 1A, and this region of the shoulders can fit in the holder portion 20c. A pair of rectangular parallelepiped projections 35 at ends on the head side of the shoulder immobilization part 30C is set at the shoulder immobilization part 30c. The distance L7 between the pair of projections 35 is set to be substantially the same as the width L8 of the occipital region immobilization part 30F. The occipital region immobilization part 30F tightly fit at ends on the shoulder side between the pair of projections 35, thereby connecting and immobilizing the shoulder immobilization part 30C and the occipital region immobilization part 30F. The thickness L9 of the projections 35 is equal to the thickness L10 that is from the lower surface of the occipital region immobilization part 30F to the bottom surface of the stepped portion 31. When the occipital region immobilization part 30F is fixed to the shoulder immobilization part 30C, the upper surface of the projections 35 and the bottom surface of the stepped portion 31 are aligned on the same level.

As shown in Figs. 18 and 19(C), the jaw immobilization part 30B has an arch shape with a downward opening and comprises an immobilization portion 36 having a holder portion 20b formed on the lower surface, and a pair of leg portions 37 on either end of the immobilization portion 36. The holder portion 20b has a shape along by the region comprising the top surface and both sides of the jaw, the lower surface of the jaw, and the top surface closer to the head and both sides of the neck of the patient 1A. These regions of the body can fit in the holder portion 20b. The distance L11 between the pair of leg portions 37 is substantially equal to the distance L7 between the pair of projections 35 of the shoulder immobilization part 30C. The shape of the bottom surface of the pair of leg portions 37 is substantially the same rectangular shape as that of the upper surface of the pair of projections 35 of the shoulder immobilization part 30C. The height L12 of each leg portion 37 is substantially equal to the thickness L13 of the neck portion 33 of the right and left temporal region immobilization parts 30D and 30E. The leg portions 37 of the jaw immobilization part 30B tightly fits each neck portion 33 of the right and left temporal region immobilization parts 30D and 30E, thereby connecting the jaw immobilization part 30B to the right and left temporal region immobilization parts 30D and 30E.

The block 3 in this embodiment is assembled according to the following procedure.

First, the end closer to the shoulders of the occipital region immobilization part 30F is tightly fit in the portion between the pair of projections 35 of the shoulder immobilization part 30C to connect the shoulder immobilization part 30C to the occipital region immobilization part 30F.

Secondly, each engagement portion 34 of the right and left temporal region immobilization parts 30D and 30E is locked on the corresponding stepped portion 31 of the occipital region immobilization part 30F, and then the jaw immobilization part 30B is attached from above across the upper open portion of the occipital region immobilization part 30F and the right and left temporal region immobilization parts 30D and 30E.

At this stage, the bottom surface of each leg portion 37 of the jaw immobilization part 30B comes in contact with the upper surface of the corresponding projection 35 of the shoulder immobilization part 30C. The inner surface of each leg portion 37 of the jaw immobilization part 30B presses each engagement portion 39 of the right and left temporal region immobilization parts 30D and 30E against the side surface 31a of each stepped portion 31 of the occipital region immobilization part 30F. This connects the jaw immobilization part 30B, the shoulder immobilization part 30C, the right and left temporal region immobilization parts 30D and 30E, and the occipital region immobilization part 30F. The parts 30B, 30C, 30D, 30E, and 30F thus form a recessed portion for tightly supporting the specific site 1, i.e., the holder portion 2.

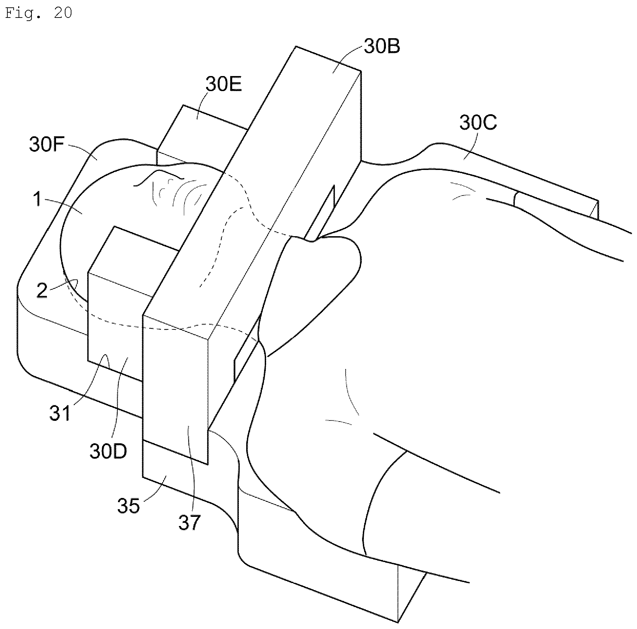

Fig. 20 shows that the head, neck, and shoulders of the patient 1A lying on their back are immobilized by the medical immobilization device 10. The lower region of the head and the lower region of the neck of the patient 1A tightly fit in the holder portion 20f of the occipital region immobilization part 30F. Both sides of the head and both sides of the neck of the patient 1A tightly fit in the holder portions 20d, 20e of the right and left temporal region immobilization parts 30D and 30E. The shoulders of the patient 1A tightly fit in the holder portion 20c of the shoulder immobilization part 30C. The jaw of the patient 1A tightly fits in the holder portion 20b of the jaw immobilization part 30B. The upper edge 2d of the portion of each holder portion 20d, 20e of the parts 30D, 30E for immobilizing the temporal region (i.e., the upper edge 2d of the portion that comes in contact with each side of the head) is located above the center in the up-and-down direction of the head. This allows the medical immobilization device 10 to firmly immobilize the head, neck, and jaw of the patient 1A with the majority of the upper portion of the block 3 being open.

In this embodiment, in particular, the jaw of the patient 1A is immobilized firmly by the jaw immobilization part 30B. This limits the movement of the patient 1A, such as shaking the head side to side, opening the mouth, or raising the chin. Additionally, because the block 3 has the upper portion open except for the part in which the jaw immobilization part 30B is present, the patient 1A is less stressed, without feeling suffocated or having an oppressive feeling. Moreover, the right and left temporal region immobilization parts 30D and 30E located closer to the shoulders of the patient 1A with the top region of the patient 1A not covered by the block 3 ensures breathability.

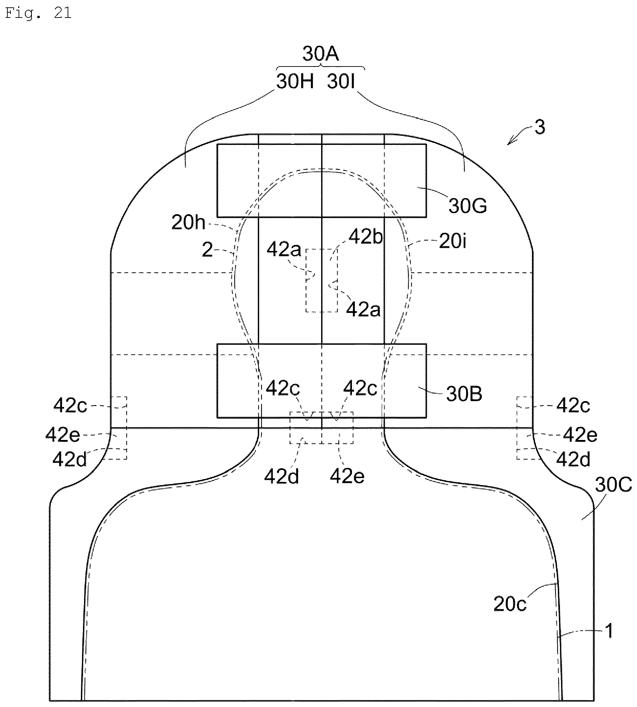

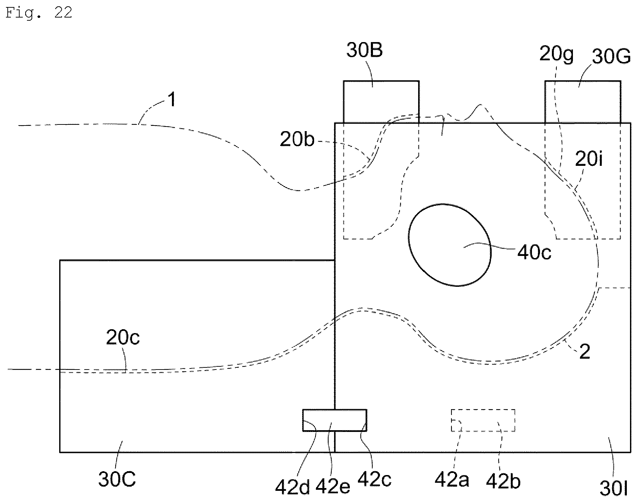

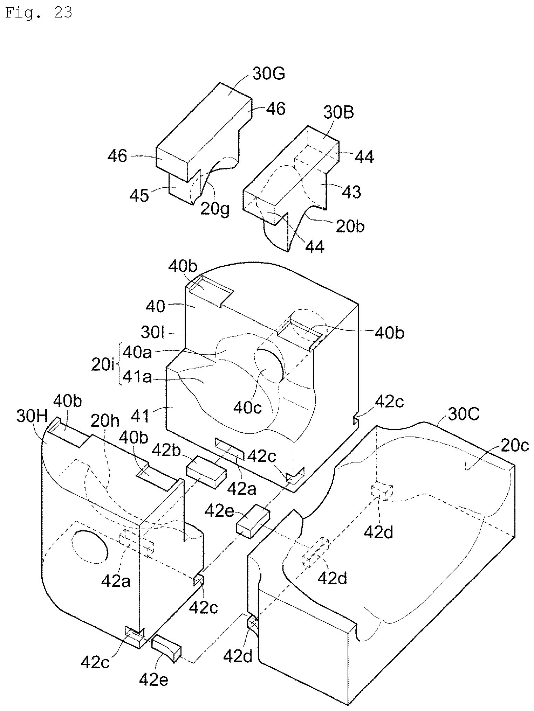

Figs. 21 to 24 show yet another embodiment. The block 3 comprises the head immobilization part 30A, the shoulder immobilization part 30C, the jaw immobilization part 30B, and a forehead immobilization part 30G that are connected to each other. The head immobilization part 30A comprises a right-side head immobilization part 30H and a left-side head immobilization part 30I that are connected to each other.

The right-side and left-side head immobilization parts 30H and 30I are each integrally formed of a side portion 40 and a lower portion 41. A holder portion 40a is formed at the inner facing surface of each side portion 40 . The holder portion 40a has a shape along by the region of the left or right side of the head of the patient 1A. This region of the head can fit in the holder portion 40a. A through-hole 40c penetrating the side portion 40 from the inner surface to the outer surface is also formed at the holder portion 40acomprises. The holder portion 41a is formed on the upper surface of each lower portion 41. The holder portion 41a has a shape along by the region of the lower surface of the left side or right side of the head of the patient 1A. This region of the head can fit in the holder portion 41a. These holder portions 40a, 41a communicate with each other to form a holder portion 20h or 20i. The right-side and left-side head immobilization parts 30H and 30I each have a symmetrical outer shape in the left-right direction. However, because the shape of the head of the patient 1A is not necessarily symmetrical, the shape of the holder portions 20h, 20i is not limited to a symmetrical shape.

The right-side and left-side head immobilization parts 30H and 30I each have a thickness greater than the thickness of the shoulder immobilization part 30C. The side portion 40 is formed at a position closer to the shoulders on the upper surface a recess 40b on which a corresponding end of the jaw immobilization part 30B is locked. A recess 40b on which an end of the forehead immobilization part 30G is locked is formed of the side portion 40 at a position closer to the head top. An engagement recess 42a is respectively formed at the mutually facing surfaces of the lower portions 41 of the parts 30H, 30I . A connecting member 42b is tightly fit into each engagement recess 42a, thereby connecting the right-side and left-side head immobilization parts 30H and 30I.

An engagement recess 42c and an engagement recess 42d are respectively formed on the surface of the right-side head immobilization part 30H facing the shoulder immobilization part 30C and on the surface of the shoulder immobilization part 30C facing the right-side head immobilization part 30H. The engagement recess 42c and the engagement recess 42d are also respectively formed on the surface of the left-side head immobilization part 30I facing the shoulder immobilization part 30C and on the surface of the shoulder immobilization part 30C facing the left-side head immobilization part 30I. The engagement recess 42c at two positions on the left and right are formed at the right-side and left-side head immobilization parts 30H and 30I. Three engagement recesses 42d corresponding to the engagement recesses 42c at the center and two positions on the left and right are formed at the shoulder immobilization part 30C.

The parts 30H, 30I, and 30C are butted against each other at their facing surfaces, and a connecting member 42e is tightly fit into each engagement recess 42c, 42d to connect the right-side head immobilization part 30H to the shoulder immobilization part 30C, and to connect the left-side head immobilization part 30I to the shoulder immobilization part 30C.

Holder portion 20c are formed on the upper surface of the shoulder immobilization part 30Ce. The holder portion 20c has a shape along by the region from the lower part to both sides of the shoulders and from the lower part closer to the shoulders to both sides of the neck of the patient 1A. This region of the shoulders and neck can fit in the holder portion 20c.

The jaw immobilization part 30B is substantially T-shaped as viewed from the shoulder side, and comprises a mating portion 43 that fits in between the right-side and left-side head immobilization parts 30H and 30I, and flange portions 44 that extend from the upper end portion of the mating portion 43 toward the left and right, and that are locked into each recess 40b on the upper surface of the right-side and left-side head immobilization parts 30H and 30I. A holder portion 20b is formed on the lower surface of the mating portion 43 . The holder portion 20b is formed in a shape along by the region from the upper surface to both sides of the jaw and the upper surface closer to the head to both sides of the neck of the patient 1A. This region of the jaw can fit in the holder portion 20b. The width of the mating portion 43 is set to be substantially equal to the distance between the side portions 40 of the right-side and left-side head immobilization parts 30H and 30I. Fitting the mating portion 43 into the part between the right-side and left-side head immobilization parts 30H and 30I allows the outer surface of the mating portion 43 and the inner surface of the head immobilization part 30A to tightly fit each other, thereby connecting the jaw immobilization part 30B to the right-side and left-side head immobilization parts 30H and 30I.

The forehead immobilization part 30G is substantially T-shaped as viewed from the shoulder side, and comprises a mating portion 45 that fits in between the right-side and left-side head immobilization parts 30H and 30I, and flange portions 46 that extend from the upper end portion of the mating portion 45 toward the left and right, and that are locked into each recess 40b on the upper surface of the right-side and left-side head immobilization parts 30H and 30I. A holder portion 20g are formed on the lower surface of the mating portion 45 . The holder portion 20g is formed in a shape along by the region from the upper surface of the forehead to the upper surface of the top of the head of the patient 1A. This region of the forehead and the top of the head can fit in the holder portion 20g. The width of the mating portion 45 is set to be substantially equal to the distance between the side portions 40 of the right-side and left-side head immobilization parts 30H and 30I. Fitting the mating portion 45 into the part between the right-side and left-side head immobilization parts 30H and 30I allows the outer surface of the mating portion 45 and the inner surface of the head immobilization part 30A to tightly fit each other, thereby connecting the forehead immobilization part 30G to the right-side and left-side head immobilization parts 30H and 30I.

With the jaw immobilization part 30B, the shoulder immobilization part 30C, the forehead immobilization part 30G, the right-side head immobilization part 30H, and the left-side head immobilization part 30I connected to each other, the holder portions 20b, 20c, 20g, 20h, and 20i of the parts 30B, 30C, 30G, 30H, and 30I form a recessed portion for tightly supporting the specific site 1, i.e., the holder portion 2.

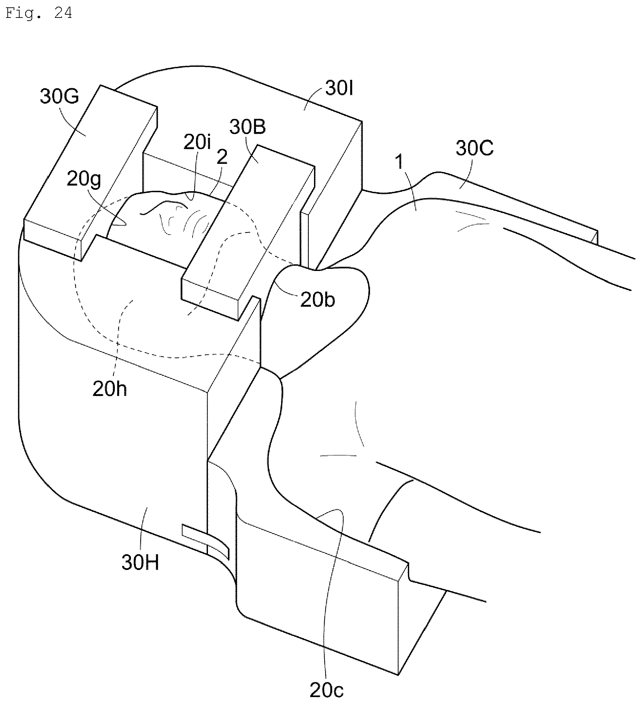

Fig. 24 shows that the head, neck, and shoulders of the patient 1A are immobilized by the medical immobilization device 10 with the patient 1A lying on their back. The region from the lower part to both sides of the head and neck of the patient 1A tightly fit in the holder portions 20h, 20i of the right-side and left-side head immobilization parts 30H and 30I. The region from the lower part to both sides of the shoulders of the patient 1A tightly fit in the holder portion 20c of the shoulder immobilization part 30C. The region from the upper surface of the jaw to the upper surface of the neck closer to the head of the patient 1A tightly fits in the holder portion 20b of the jaw immobilization part 30B.
The region from the upper surface of the forehead to the upper surface of the top of the head of the patient 1A tightly fits in the holder portion 20g of the forehead immobilization part 30G. The upper edge 2d of each holder portion 20h, 20i (i.e., the upper edge 2d of the portion that comes in contact with each side of the patient's head) is located above the center in the up-and-down direction of the head. This allows the medical immobilization device 10 to firmly immobilize the head, neck, jaw, and forehead of the patient 1A with the upper portion of the block 3 being partly open.

In the embodiment above, in particular, the jaw and the forehead of the patient 1A are immobilized firmly by the jaw immobilization part 30B and the forehead immobilization part 30G. This limits the movement of the patient 1A, such as shaking the head side to side, opening the mouth, or raising the chin. Additionally, because the block 3 has the upper portion open except for the part in which the jaw immobilization part 30B and the forehead immobilization part 30G are present, the patient 1A does not feel suffocated or have an oppressive feeling.

In the embodiment above, the region from the head to the shoulders of the patient 1A is determined to be the specific site 1, and at least the region from the lower part to both sides of the specific site 1, preferably a region beyond this region, is set to fit in the holder 2. However, the medical immobilization device 10 of the present invention is not limited to this embodiment. For example, other regions of the patient 1A or the entire body may be set to be a specific site 1, and the medical immobilization device 10 may be structured such that at least the region from the lower part to both sides of the specific site 1, preferably a region beyond this region, fit in the holder 2.

### Description of the Reference Numerals

1 specific site
1A patient
2 holder portion
2a to 2c holder portion
3 block
3A right-side head immobilization part
3B left-side head immobilization part
3C shoulder immobilization part
4 connector
5 ventilation portion
6 manufacturing immobilization device support equipment
10 medical immobilization device
30A head immobilization part
30B jaw immobilization part
30C shoulder immobilization part
30G forehead immobilization part
20b to 20i holder portion
100 radiotherapy equipment
101 support table

## Claims

1. A medical immobilization device (10) for immobilizing a specific site (1) of a body of a patient (1A) with the patient (1A) lying on his or her back,
the medical immobilization device (10) comprising a block (3) made of a material that allows radiation to pass through,
the block (3) comprising a holder portion (2, 2a - 2c, 20b - 20i) in which a region including a lower surface and both sides continuous with the lower surface of the specific site (1) tightly fits,
the block (3) having all or a part of an upper side thereof open, wherein
the block (3) comprises a plurality of parts (3A, 3B, 30A - 30G) that are connected to each other,
the block (3) comprises a head immobilization part (30A) for immobilizing a head, a shoulder immobilization part (30C) for immobilizing shoulders, and **characterized in that** it further comprises at least either a jaw immobilization part (30B) for immobilizing a jaw or a forehead immobilization part (30G) for immobilizing a forehead, or both,
the head immobilization part (30A), the shoulder immobilization part (30C), and at least either the jaw immobilization part (30B) for immobilizing a jaw or the forehead immobilization part (30G) for immobilizing a forehead, or both are connected to each other,
the head immobilization part (30A) comprises at least a right-side head immobilization part (3A) for immobilizing a right-side of the head and a left-side head immobilization part (3B) for immobilizing a left-side of the head, and
at least either the jaw immobilization part (30B) or the forehead immobilization part (30G), or both are connected at each end to the right-side head immobilization part (3A) and the left-side head immobilization part (3B) across an upper open portion between the right-side head immobilization part (3A) and the left-side head immobilization part (3B).

2. The medical immobilization device (10) according to claim 1, wherein an upper edge (2d) of a portion of the holder portion (2, 2a - 2c, 20b - 20i) that comes into contact with the sides of the specific site (1) is located above the center of the specific site (1) in an up-and-down direction.

3. The medical immobilization device (10) according to claim 1 or 2, wherein the region from the lower surface to both sides of the specific site (1) fits in the holder portion (20b - 20i) with the entire surface of the region being tightly in contact.

4. The medical immobilization device (10) according to any one of claims 1 to 3, wherein the holder portion (20b - 20i) comprises a ventilation portion (5) with which the specific site (1) does not come into contact, and the region from the lower surface to both sides of the specific site (1) tightly fits in the holder portion (20b - 20i) excluding the ventilation portion (5).

5. The medical immobilization device (10) according to claim 1, wherein
the block (3) comprises the head immobilization part (30A), the shoulder immobilization part (30C), and the jaw immobilization part (30B),
the head immobilization part (30A), the shoulder immobilization part (30C), and the jaw immobilization part (30B) being connected to each other, the head immobilization part (30A) comprises a right temporal region immobilization part (30D) for immobilizing a right temporal region, a left temporal region immobilization part (30D) for immobilizing a left temporal region, and an occipital region immobilization part (30F) for immobilizing an occipital region,
the occipital region immobilization part (30F) and the shoulder immobilization part (30C) are connected in a longitudinal direction,
the left temporal region immobilization part (30D) and the right temporal region immobilization part (30D) are respectively connected to a left side and a right side of the occipital region immobilization part (30F), and
the jaw immobilization part (30B) is connected at each end to the left temporal region immobilization part (30E) and the right temporal region immobilization part (30D) across an upper open portion between the left temporal region immobilization part (30E) and the right temporal region immobilization part (30D).

6. The medical immobilization device (10) according to claim 1, wherein
the block (3) comprises the head immobilization part (30A), the shoulder immobilization part (30C), the jaw immobilization part (30B), and the forehead immobilization part (30G), the head immobilization part (30A), the shoulder immobilization part (30C), the jaw immobilization part (30B), and the forehead immobilization part (30G) being connected to each other,
the head immobilization part (30A) and the shoulder immobilization part (30C) are connected in a longitudinal direction, and
the jaw immobilization part and the forehead immobilization part (30G) are connected at each end to the left-side head immobilization part (3B) and the right head immobilization part (30A) across the upper open portion between the right-side head immobilization part (3A) and the left-side head immobilization part (3B).

## Patentansprüche

1. Medizinische Immobilisierungsvorrichtung (10) zum Immobilisieren einer spezifischen Stelle (1) eines Körpers eines Patienten (1A), wobei der Patient (1A) auf seinem Rücken liegt,
wobei die medizinische Immobilisierungsvorrichtung (10) einen Block (3) umfasst, der aus einem Material hergestellt ist, das Strahlung durchlässt,
wobei der Block (3) einen Halterabschnitt (2, 2a - 2c, 20b - 20i) umfasst, in den ein Bereich, der eine untere Oberfläche und beide Seiten umfasst, die mit der unteren Oberfläche der spezifischen Stelle (1) zusammenhängen, eng passt,
wobei der Block (3) die gesamte oder einen Teil einer oberen Seite davon offen aufweist,
wobei der Block (3) eine Vielzahl von Teilen (3A, 3B, 30A - 30G) umfasst, die miteinander verbunden sind,
der Block (3) einen Kopfimmobilisierungsteil (30A) zum Immobilisieren eines Kopfes, einen Schulterimmobilisierungsteil (30C) zum Immobilisieren von Schultern umfasst, und **dadurch gekennzeichnet, dass** sie ferner mindestens entweder einen Kieferimmobilisierungsteil (30B) zum Immobilisieren eines Kiefers oder einen Stirnimmobilisierungsteil (30G) zum Immobilisieren einer Stirn oder beides umfasst,
wobei der Kopfimmobilisierungsteil (30A), der Schulterimmobilisierungsteil (30C),
und mindestens entweder der Kieferimmobilisierungsteil (30B) zum Immobilisieren eines Kiefers oder der Stirnimmobilisierungsteil (30G) zum Immobilisieren einer Stirn oder beide miteinander verbunden sind,
der Kopfimmobilisierungsteil (30A) mindestens einen rechtsseitigen Kopfimmobilisierungsteil (3A) zum Immobilisieren einer rechten Seite des Kopfes und einen linksseitigen Kopfimmobilisierungsteil (3B) zum Immobilisieren einer linken Seite des Kopfes umfasst, und
mindestens entweder der Kieferimmobilisierungsteil (30B) oder der Stirnimmobilisierungsteil (30G) oder beide an jedem Ende mit dem rechtsseitigen Kopfimmobilisierungsteil (3A) und dem linksseitigen Kopfimmobilisierungsteil (3B) über einen oberen offenen Abschnitt zwischen dem rechtsseitigen Kopfimmobilisierungsteil (3A) und dem linksseitigen Kopfimmobilisierungsteil (3B) verbunden sind.

2. Medizinische Immobilisierungsvorrichtung (10) nach Anspruch 1, wobei sich ein oberer Rand (2d) eines Abschnitts des Halterabschnitts (2, 2a - 2c, 20b - 20i), der mit den Seiten der spezifischen Stelle (1) in Kontakt kommt, über der Mitte der spezifischen Stelle (1) in einer Aufwärts-und-Abwärts-Richtung befindet.

3. Medizinische Immobilisierungsvorrichtung (10) nach Anspruch 1 oder 2, wobei der Bereich von der unteren Oberfläche zu beiden Seiten der spezifischen Stelle (1) in den Halterabschnitt (20b - 20i) passt, wobei die gesamte Oberfläche des Bereichs eng in Kontakt ist.

4. Medizinische Immobilisierungsvorrichtung (10) nach einem der Ansprüche 1 bis 3, wobei der Halterabschnitt (20b - 20i) einen Belüftungsabschnitt (5) umfasst, mit dem die spezifische Stelle (1) nicht in Kontakt kommt, und der Bereich von der unteren Oberfläche zu beiden Seiten der spezifischen Stelle (1) in den Halterabschnitt (20b - 20i) mit Ausnahme des Belüftungsabschnitts (5) eng passt.

5. Medizinische Immobilisierungsvorrichtung (10) nach Anspruch 1, wobei der Block (3) den Kopfimmobilisierungsteil (30A), den Schulterimmobilisierungsteil (30C) und den Kieferimmobilisierungsteil (30B) umfasst,
wobei der Kopfimmobilisierungsteil (30A), der Schulterimmobilisierungsteil (30C),
und der Kieferimmobilisierungsteil (30B) miteinander verbunden sind, wobei der Kopfimmobilisierungsteil (30A) einen rechten Schläfenbereichsimmobilisierungsteil (30D) zum Immobilisieren eines rechten Schläfenbereichs, einen linken Schläfenbereichsimmobilisierungsteil (30D) zum Immobilisieren eines linken Schläfenbereichs und einen Hinterhauptbereichsimmobilisierungsteil (30F) zum Immobilisieren eines Hinterhauptbereichs umfasst,
wobei der Hinterhauptbereichsimmobilisierungsteil (30F) und der Schulterimmobilisierungsteil (30C) in einer Längsrichtung verbunden sind,
wobei der linke Schläfenbereichsimmobilisierungsteil (30D) und der rechte Schläfenbereichsimmobilisierungsteil (30D) jeweils mit einer linken Seite und einer rechten Seite des Hinterhauptbereichsimmobilisierungsteils (30F) verbunden sind, und
der Kieferimmobilisierungsteil (30B) an jedem Ende mit dem linken Schläfenbereichsimmobilisierungsteil (30E) und dem rechten Schläfenbereichsimmobilisierungsteil (30D) über einen oberen offenen Abschnitt zwischen dem linken Schläfenbereichsimmobilisierungsteil (30E) und dem rechten Schläfenbereichsimmobilisierungsteil (30D) verbunden ist.

6. Medizinische Immobilisierungsvorrichtung (10) nach Anspruch 1, wobei der Block (3) den Kopfimmobilisierungsteil (30A), den Schulterimmobilisierungsteil (30C), den Kieferimmobilisierungsteil (30B) und den Stirnimmobilisierungsteil (30G) umfasst, wobei der Kopfimmobilisierungsteil (30A), der Schulterimmobilisierungsteil (30C), der Kieferimmobilisierungsteil (30B) und der Stirnimmobilisierungsteil (30G) miteinander verbunden sind,
wobei der Kopfimmobilisierungsteil (30A) und der Schulterimmobilisierungsteil (30C) in einer Längsrichtung verbunden sind, und
der Kieferimmobilisierungsteil und der Stirnimmobilisierungsteil (30G) an jedem Ende mit dem linksseitigen Kopfimmobilisierungsteil (3B) und dem rechten Kopfimmobilisierungsteil (30A) über den oberen offenen Abschnitt zwischen dem rechtsseitigen Kopfimmobilisierungsteil (3A) und dem linksseitigen Kopfimmobilisierungsteil (3B) verbunden sind.

## Revendications

1. Dispositif médical d'immobilisation (10) destiné à immobiliser un site spécifique (1) d'un corps d'un patient (1A), le patient (1A) étant allongé sur le dos,
le dispositif médical d'immobilisation (10) comprenant un bloc (3) fait dans un matériau qui permet le passage d'un rayonnement,
le bloc (3) comprenant une portion de support (2, 2a - 2c, 20b - 20i) dans laquelle une région incluant une surface inférieure et les deux côtés en prolongement avec la surface intérieure du site spécifique (1) s'adapte parfaitement,
le bloc (3) ayant la totalité ou une partie d'un côté supérieur de celui-ci ouverte, dans lequel
le bloc (3) comprend une pluralité de parties (3A, 3B, 30A - 30G) qui sont connectées les unes aux autres,
le bloc (3) comprend une partie d'immobilisation de tête (30A) pour immobiliser une tête, une partie d'immobilisation d'épaules (30C) pour immobiliser des épaules, et
**caractérisé en ce qu'**il comprend en outre au moins l'une ou l'autre d'une partie d'immobilisation de mâchoire (30B) pour immobiliser une mâchoire ou d'une partie d'immobilisation de front (30G) pour immobiliser un front, ou les deux,
la partie d'immobilisation de tête (30A), la partie d'immobilisation d'épaules (30C), et au moins l'une ou l'autre de la partie d'immobilisation de mâchoire (30B) pour immobiliser une mâchoire ou la partie d'immobilisation de front (30G) pour immobiliser un front, ou les deux, sont connectées les unes aux autres,
la partie d'immobilisation de tête (30A) comprend au moins une partie d'immobilisation de tête côté droit (3A) pour immobiliser un côté droit de la tête et une partie d'immobilisation de tête côté gauche (3B) pour immobiliser un côté gauche de la tête, et
au moins l'une ou l'autre de la partie d'immobilisation de mâchoire (30B) et de la partie d'immobilisation de front (30G), ou les deux, sont connectées au niveau de chaque extrémité à la partie d'immobilisation de tête côté droit (3A) et à la partie d'immobilisation de tête côté gauche (3B) à travers une portion ouverte supérieure entre la partie d'immobilisation de tête côté droit (3A) et la partie d'immobilisation de tête côté gauche (3B).

2. Dispositif médical d'immobilisation (10) selon la revendication 1, dans lequel un bord supérieur (2d) d'une portion de la portion de support (2, 2a - 2c, 20b - 20i) qui vient en contact avec les côtés du site spécifique (1) est situé au-dessus du centre du site spécifique (1) dans une direction haut/bas.

3. Dispositif médical d'immobilisation (10) selon la revendication 1 ou 2, dans lequel la région depuis la surface inférieure jusqu'aux deux côtés du site spécifique (1) s'adapte dans la portion de support (20b - 20i), la surface entière de la région étant contact serré.

4. Dispositif médical d'immobilisation (10) selon l'une quelconque des revendications 1 à 3, dans lequel la portion de support (20b - 20i) comprend une portion de ventilation (5) avec laquelle le site spécifique (1) ne vient pas en contact, et la région depuis la surface inférieure jusqu'aux deux côtés du site spécifique (1) s'adapte parfaitement dans la portion de support (20b - 20i), excluant la portion de ventilation (5).

5. Dispositif médical d'immobilisation (10) selon la revendication 1, dans lequel
le bloc (3) comprend la partie d'immobilisation de tête (30A), la partie d'immobilisation d'épaules (30C) et la partie d'immobilisation de mâchoire (30B),
la partie d'immobilisation de tête (30A), la partie d'immobilisation d'épaules (30C), et la partie d'immobilisation de mâchoire (30B) étant connectées les unes aux autres, la partie d'immobilisation de tête (30A) comprenant une région temporale droite (30D) pour immobiliser une région temporale de droite, une partie d'immobilisation de région temporale gauche (30D) pour immobiliser une région temporale de gauche, et une partie d'immobilisation de région occipitale (30F) pour immobiliser une région occipitale,
la partie d'immobilisation de région occipitale (30F) et la partie d'immobilisation d'épaules (30C) sont connectées dans une direction longitudinale,
la partie d'immobilisation de région temporale gauche (30D) et la partie d'immobilisation de région temporale droite (30D) sont connectées respectivement à un côté gauche et à un côté droit de la partie d'immobilisation de région occipitale (30F), et
la partie d'immobilisation de mâchoire (30B) est connectée au niveau de chaque extrémité à la partie d'immobilisation de région temporale gauche (30E) et à la partie d'immobilisation de la région temporale droite (30D) à travers une portion ouverte supérieure entre la partie d'immobilisation de région temporale gauche (30E) et la partie d'immobilisation de région temporale droite (30D).

6. Dispositif médical d'immobilisation (10) selon la revendication 1, dans lequel
le bloc (3) comprend la partie d'immobilisation de tête (30A), la partie d'immobilisation d'épaules (30C), la partie d'immobilisation de mâchoire (30B), et la partie d'immobilisation de front (30G), la partie d'immobilisation de tête (30A), la partie d'immobilisation d'épaules (30C), la partie d'immobilisation de mâchoire (30B), et la partie d'immobilisation de front (30G) étant connectées les unes aux autres,
la partie d'immobilisation de tête (30A) et la partie d'immobilisation d'épaules (30C) sont connectées dans une direction longitudinale, et
la partie d'immobilisation de mâchoire et la partie d'immobilisation de front (30G) sont connectées au niveau de chaque extrémité à la partie d'immobilisation de tête côté gauche (3B) et à la partie d'immobilisation de tête côté droit (30A) à travers la portion ouverte supérieure entre la partie d'immobilisation de tête côté droit (3A) et la partie d'immobilisation de tête côté gauche (3B).
